# EUROPEAN PATENT APPLICATION

(11) **EP 3 747 886 A1**
(43) Date of publication of application: **09.12.2020**
(21) Application number: 20178381.8
(22) Date of filing: 04.06.2020
(51) Int. Cl.: C07D 471/04

(54) **DERIVATIVES OF HYDROCINCHONINE, HYDROQUININE AND HYDROQUINIDINE, METHOD FOR THE PREPARATION THEREOF AND USE THEREOF AS CATALYSTS OF PTC PROCESSES**

(30) Priority: 04.06.2019 PL 43014119; 04.06.2019 PL 43014219
(71) Applicant: Instytut Chemii Organicznej Polskiej Akademii Nauk, 01-224 Warszawa (PL)
(72) Inventor: Jurczak, Janusz, 02-384 Warszawa (PL); Majdecki, Maciej, 05-090 Raszyn (PL); Niedbala, Patryk, 05-090 Raszyn (PL)
(74) Representative: LDS Lazewski Depo & Partners

(57) **Abstract**

The invention relates to the hydrocinchonidine, hydrocinchonine, hydroquinine and hydroquinidine derivatives of general Formula 1. The invention also relates to a method for preparation of the compounds of the invention defined by general Formula 1, as well as to their use in asymmetric organic chemistry under phase transfer (PTC) conditions.

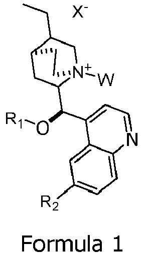

## Description

The invention relates to hydrocinchonidine, hydrocinchonine, hydroquinine and hydroquinidine derivatives, method for preparation thereof and use thereof in asymmetric organic chemistry reactions. The compounds of the invention are useful as catalysts in Phase-Transfer Catalysis (PTC) reactions, both in the liquid-liquid and liquid-solid variants.

The compounds according to the invention are based on the quinuclidine backbone. Quinuclidine is a structural element found in cinchonidine, cinchonine, quinine and quinidine. One of the common features of the mentioned alkaloids is the presence of a vinyl substituent (C10-C11 double bond) attached to the quinuclidine.

One of the objects of the present invention is to provide such cinchona alkaloids derivatives wherein said vinyl substituent is converted to an alkyl moiety by reducing the double bond. As a result, derivatives of cinchona alkaloids are obtained, such as hydrocinchonidine, hydrocinchonine, hydroquinine and hydroquinidine. These compounds, after further functionalizations, can be used as catalysts in PTC reactions, in particular in asymmetric PTC reactions.

Hydrocynchonidine derivatives and related compounds, which are catalysts in asymmetric reactions (PTC), are known in the art. Nevertheless, the synthetic possibilities inherent in the reactions carried out under phase transfer conditions mean that new catalysts of this type are constantly being searched for. In particular, catalysts containing groups changing the electron density in the aromatic ring in their structure are desirable.

A significant number of chiral catalysts using the structure of *Cinchona* alkaloids, such as cinchonine, cinchonidine, quinine or quinidine (Formula A), were presented as early as the 1980s (Dolling, U.-H., et al., J. Am. Chem. Soc., 1984, 106, 446; Dolling, U.-H., et al., J. Org. Chem., 1987, 52, 4745).

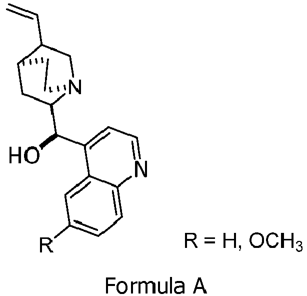

Simple nitrogen-substituted *Cinchona* alkaloids are known in the art and have found applications in PTC reactions (Formula B). However, they did not show satisfactory catalytic activities, despite their use even in the amount of 10 mol% relative to the substrate, which was manifested mainly by low enantiomeric excesses (O'Donnell, Wu, S., Huffman, J.C., Tetrahedron, 1994, 50, 4507). This concept was then continued by the Lygo group (Lygo, B., Wainwright, P.G., Tetrahedron, 1998, 55, 6289; Lygo, B., Crosby, J., Peterson, J.A., Tetrahedron Letters, 1999, 40, 8671), using compounds having an additional substituted hydroxyl group. Moreover, these catalysts were characterized by the presence of a reduced C10-C11 bond (Formula C).

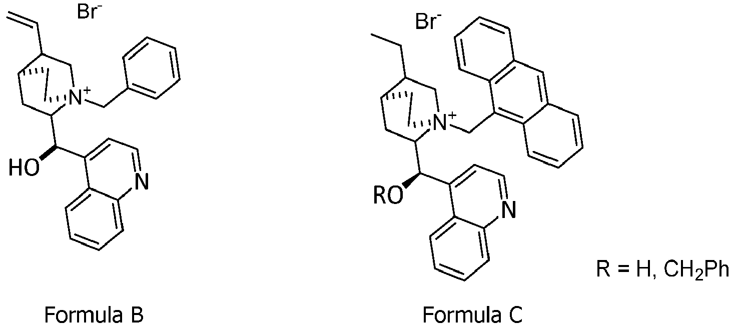

In order to improve the enantioselectivity of the conducted reactions, a class of dimeric catalysts with high catalytic activity was also synthesized and used (Jew, S.-S., Park, H.-G., et al., Tetrahedron Lett., 2001, 42, 4645) (Formula D). Nevertheless, the mentioned derivatives are characterized by a significant molecular weight, which translates into their higher consumption during the enantioselective reaction. In state of the art there are also reports about other hydrocinchonidine derivatives used in alkylation reactions containing in their structure an aromatic substituent on the nitrogen atom and an appropriately substituted hydroxyl group. Among them, catalysts with additional groups influencing the electron density of the ring at the nitrogen substituent are presented (Formula E) (Yoo, M.-S., Jeong, B.-S., Lee, J.-H., Park, H., Jew, S., Org. Lett., 2005, 7, 1129).

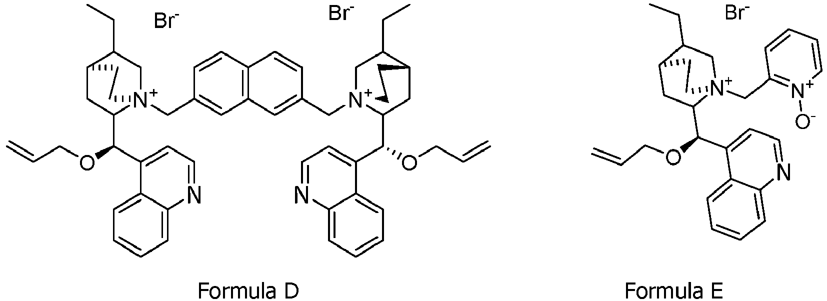

The object of the present invention is to provide chemical compounds being variously substituted hydrocinchonidine, hydrocinchonine, hydroquinine and hydroquinidine derivatives (i.e., cinchona alkaloid derivatives containing a reduced C10-C11 double bond attached to a quinuclidine ring) that are suitable for use as catalysts in an asymmetric synthesis carried out under phase transfer conditions. These compounds can be readily obtained from commercially available, cheap starting materials. Moreover, these catalysts can be used at low concentrations (10 mol% or less). Both the catalyst synthesis and the subsequent reaction under PTC conditions can be carried out in a continuous flow.

In particular, a further object is to provide amide derivatives of hydrocinchonidine, hydrocinchonine, hydroquinine and hydroquinidine. Such compounds are used as catalysts in liquid-liquid or liquid-solid phase transfer reactions, leading to a high reaction yield and high enantiomeric excess.

In particular, a further object is to provide uniformly *N,O*-disubstituted derivatives of hydrocinchonidine, hydrocinchonine, hydroquinine and hydroquinidine. The distinguishing feature of the *N,O*-disubstituted derivatives according to the invention is their very efficient synthesis due to the possibility of using the "one-pot" technique or generating *in situ,* without the need to separate the catalyst from the reaction mixture.

In particular, a further object is to provide a method for the preparation of hydrocinchonidine, hydrocinchonine, hydroquinine and hydroquinidine derivatives which has been solved by the novel derivatives of general Formula 1.

In a first aspect, the invention provides a compound of general Formula 1: 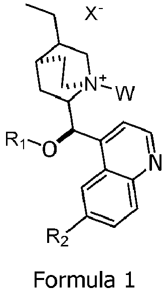 wherein:
**R₁** is independently hydrogen, C₁-C₅ alkyl, C₅-C₁₆ aryl, or substituted benzyl of formula **A,** wherein A is: 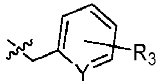 and **R₃** is independently hydrogen, halogen, trifluoromethyl (-CF₃), nitrile (-CN) or nitro (-NO₂) group and **Y** is any heteroatom or CH group;
**R₂** is independently hydrogen or methoxy group (-OCH₃);
**X⁻** is any inorganic anion;
**W** is said **A,** wherein **Y** and **R₃** are as above; or **W** is 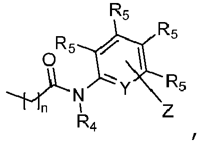 wherein **n** is an integer ranging from 1 to 5; **R₄** is independently hydrogen, C₁-C₅ alkyl, or C₅-C₁₆ aryl; **R₅** is independently hydrogen, halogen, C₁-C₁₂ alkyl, C₅-C₁₆ aryl, C₁-C₅ branched alkyl, trifluoromethyl (-CF₃), nitrile (-CN), nitro (-NO2), alkoxy -OC₁-C₁₂ group or **Z** of the formula 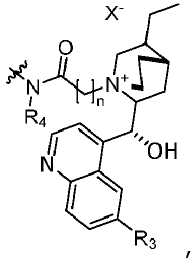 wherein **Y, n, R₃, R4** and **X** are as above.

In one embodiment, the compound of Formula 1 is selected from:

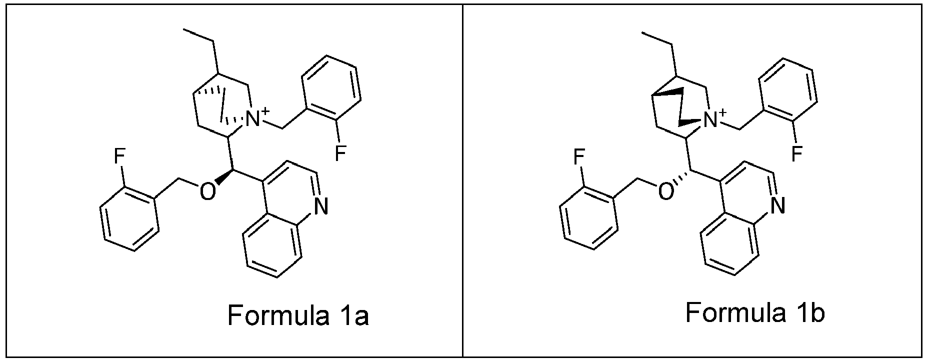

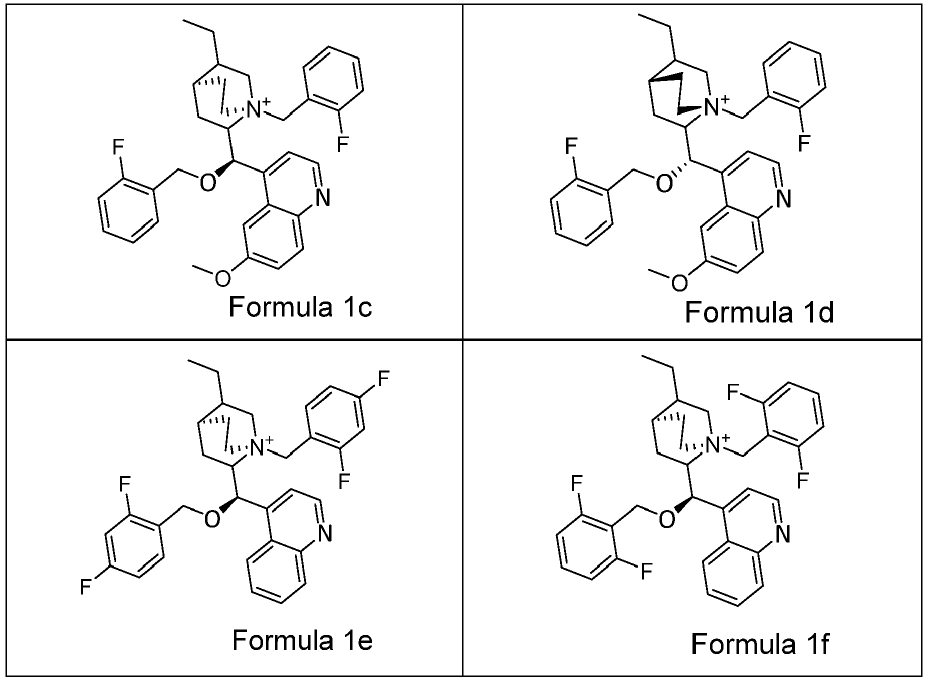

In another embodiment, the compound of Formula 1 is selected from:

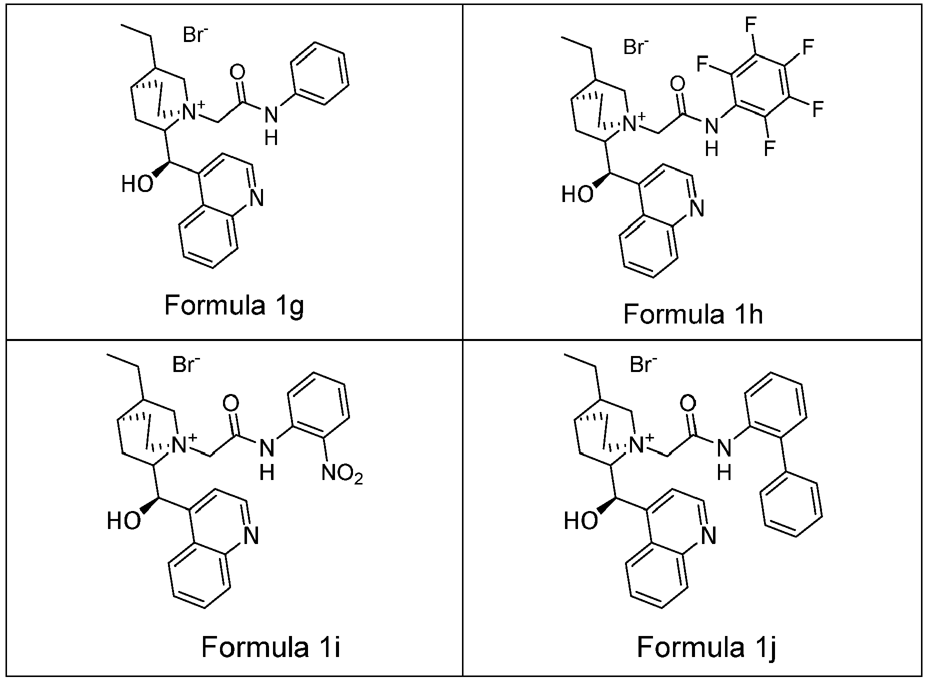

In another embodiment, the compound of Formula 1 is:

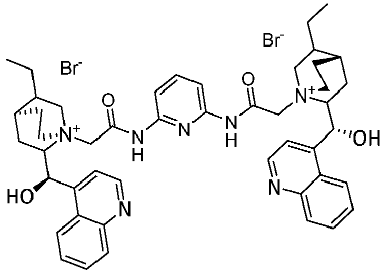

In another embodiment, the invention relates to a compound of general Formula 1 wherein when **R₁** is hydrogen, C₁-C₅ alkyl, C₅-C₁₆ aryl, then **W** is 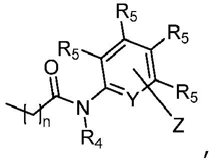 wherein **n, R₄** and **Z,** if present, are as above.

In another embodiment, the invention relates to a compound of general Formula 1 wherein when **R₁** is said **A, W** is also said **A,** wherein **Y** and **R₃** are as above.

In a second aspect, the present invention relates to a method for preparation of a compound of Formula 1, in particular a compound selected from the group of compounds of Formulas 1a to 1f, the method comprising reacting two molar equivalents of a compound of formula: 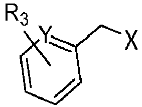 wherein:
**R₃** is a halogen atom (-F, -CI, -Br or -I), a trifluoromethyl (-CF₃), nitrile (-CN) or nitro (-NO₂) group;
**Y** is any heteroatom;
**X** is Cl or Br;
with a compound selected from the group of hydrocinchonidine, hydrocinchonine, hydroquinine or hydroquinidine, wherein the reaction is performed for 1 to 5 hours at room temperature in a two-phase system using dichloromethane as an aprotic solvent.

In one embodiment, the method for preparation of a compound of Formula 1, in particular a compound selected from the group of compounds of Formulas 1g to 1j, comprises reacting no more than two molar equivalents of the compound of Formula: 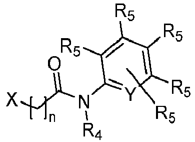 wherein:
**n** is an integer ranging from 1 to 5;
**R₄** is independently hydrogen, C₁-C₅ alkyl, or C₅-C₁₆ aryl;
**R₅** is independently hydrogen, halogen, C₁-C₁₂ alkyl, C₅-C₁₆ aryl, C₁-C₅ branched alkyl, nitro (-NO₂), trifluoromethyl (-CF₃), nitrile (-CN), alkoxy -OC₁-C₁₂ group;
**X** is Cl or Br;
**Y** is a nitrogen atom or a CH group;
with a compound selected from the group of hydrocinchonidine, hydrocinchonine, hydroquinine or hydroquinidine, wherein the reaction is performed for 1 to 5 hours in an aprotic solvent at reflux temperature.

In another embodiment of the method for preparation of a compound of Formula 1, in particular a compound of Formula 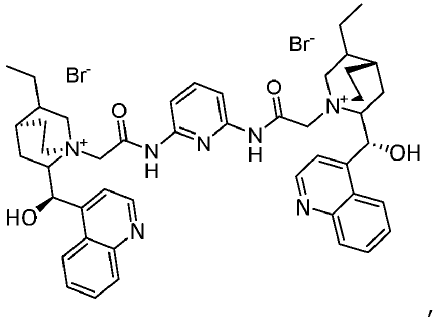 the said method comprises reacting no more than one molar equivalent of a compound of formula: 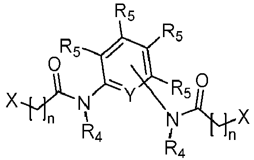 wherein:
**n** is an integer ranging from 1 to 5;
**R4** is independently hydrogen, C₁-C₅ alkyl, or C₅-C₁₆ aryl;
**R₅** is independently hydrogen, halogen, C₁-C₁₂ alkyl, C₅-C₁₆ aryl, C₁-C₅ branched alkyl, nitro (-NO₂), trifluoromethyl (-CF₃), nitrile (-CN), alkoxy -OC₁-C₁₂ group;
**X** is Cl or Br;
**Y** is a nitrogen atom or a CH group;
with a compound selected from the group of hydrocinchonidine, hydrocinchonine, hydroquinine or hydroquinidine, wherein the reaction is performed for 1 to 5 hours in an aprotic solvent at reflux temperature.

In a third aspect, the present invention relates to the use of a compound of Formula 1, in particular a compound selected from the group of compounds of Formulas 1a to 1j and a compound of Formula 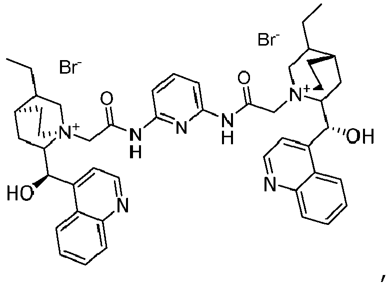 as a catalyst in an asymmetric phase transfer catalysis (PTC).

In one embodiment of the present invention, said asymmetric reaction is an asymmetric Schiff base alkylation reaction, an asymmetric epoxidation reaction or an asymmetric Michael addition reaction.

In another embodiment of the present invention, said asymmetric Michael addition reaction is an asymmetric cyanation reaction.

In another embodiment of the present invention, said asymmetric alkylation reaction of the Schiff bases proceeds with α-amino acid imine ester derivatives as substrates.

In another embodiment of the present invention, the reaction takes place under liquid-liquid or liquid-solid phase transfer conditions.

In another embodiment of the present invention, the reaction is carried out under flow conditions.

In yet another embodiment, the present invention relates to the use of a compound represented by general Formula 1, in particular a compound selected from the group of compounds of Formulas 1a-1f, as a catalyst in an asymmetric phase transfer reaction (PTC) wherein the catalyst is generated *in situ* or catalyzed reaction is carried out using the one-pot technique.

The subject of the invention is shown in the drawing, where:
- Fig. 1 shows a visualization of the system for a continuous-flow reaction using an *N*,*O-*disubstituted derivative according to the invention,
- Fig. 2 shows the HPLC chromatogram of the reaction mixture without purification for the reaction using the *N,O*-disubstituted derivative according to the invention.

All technical and scientific terms used in this document have the meaning as commonly understood by one skilled in the art.

The compounds disclosed herein have carbon stereogenic centers. Such compounds include racemic mixtures of all stereoisomers, including enanantiomers, diastereoisomers and atropisomers. Both racemic and diastereomeric mixtures and all individual optical isomers, isolated and synthesized, substantially optically pure, are within the scope of the present invention.

The invention includes all any stereochemical forms, including enantiomeric or diastereomeric, and geometric isomers of the compounds disclosed herein, or mixtures thereof.

Salts of the compounds of the invention as used herein include salts with any inorganic anion. Inorganic anions are common anions used in the art and are known to those skilled in the art. The preferred inorganic anions are the halides, i.e. fluoride, chloride, bromide or iodide anion.

The solvent used in the method for preparation of the compounds of the invention is an aprotic solvent. Preferably the solvent is selected from the group comprising tetrahydrofuran (THF) and/or dichloromethane.

The *N,O*-disubstituted derivatives of the invention can be prepared by the "one-pot" technique. This implies a synthetic approach in which the reactant is subjected to subsequent chemical reactions successively in one reactor. This avoids the purification process of intermediate chemicals, while increasing the yield. For example, the one-pot technique within the meaning of the present invention consists in adding to a solution hydrocinchonidine or an analog thereof of the appropriate benzyl halide and base solution. After a proper time has elapsed, the appropriate reagents (depending on the reaction to be catalyzed) and, for example, a 50% KOH solution are added to the catalyst system thus prepared.

The *N,O*-disubstituted derivatives according to the invention can also be prepared in situ without the need for the catalyst to be separated from the reaction mixture and then used as a catalyst in the reaction as such. For example, after generating the catalyst, its solution is used in the subsequent selected asymmetric reaction.

The compounds of the invention find use as catalysts in reactions carried out under flow conditions.

In one example, by flow conditions is meant catalyst synthesis by mixing a solution of hydrocinchonidine or an analogue thereof in a appropriate solvent and a appropriate benzyl halide and 10% base solution using constant flow of the organic and aqueous phases. After a proper time has elapsed, a mixture of the substrate solution and the appropriate alkylating agent in an organic solvent and, for example, a 50% KOH solution are introduced into the catalyst system in a continuous flow.

In another example, by flow conditions is meant the introduction into the reaction system of a suitably prepared catalyst solution, substrate, and an appropriate alkylating agent in an organic solvent, and, for example, a 50% KOH solution.

Both the synthesis of the compounds of the invention and their use in the subsequent reaction under PTC conditions can be carried out in a continuous flow.

The compounds of the present invention find use as catalysts in asymmetric reactions under PTC conditions. In particular, said reaction may be an asymmetric Schiff base alkylation reaction, an asymmetric epoxidation reaction, and an asymmetric Michael addition reaction. Preferably, the asymmetric Michael addition is a cyanation reaction.

The compounds according to the invention can be used as catalysts by using their low concentrations. In particular, the compounds of the invention are used as catalysts in an amount of 10% or less, preferably 5% or less, more preferably 3% or less.

### Examples

The subject of the invention is shown in the following examples in Table 1, which shows representative structures of the compounds of the invention along with their spectral characteristics (mass spectrometry). The following compounds of the invention do not limit the scope of the invention as defined in the claims, but are merely representative embodiments of the invention.

**Table 1.**

| | |
|---|---|
| 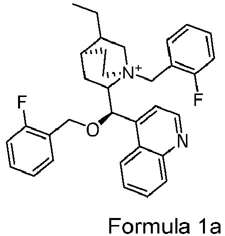 | 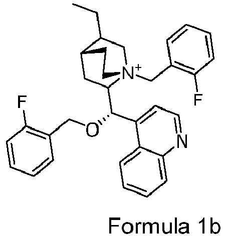 |
| HRMS ESI (m/z): | HRMS ESI (m/z): |
| calc. for C₃₃H₃₅F₂N₂O [M]⁺: 513.2717, | calc. for C₃₃H₃₅F₂N₂O [M]⁺: 513.2717, |
| found: 513.2710. | found: 513.2706. |
| 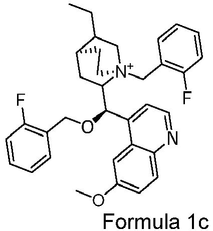 | 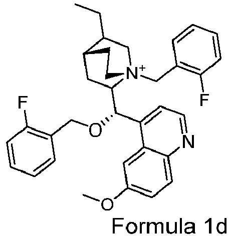 |
| HRMS ESI (m/z): | HRMS ESI (m/z): |
| calc. for C₃₄H₃₇F₂N₂O₂ [M]⁺: 543.2823, | calc for C₃₄H₃₇F₂N₂O₂ [M]⁺: 543.2823, |
| found: 543.2803. | found: 543.2811. |
| 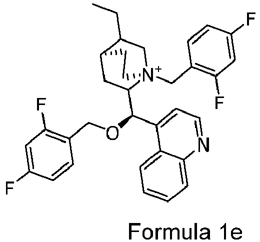 | 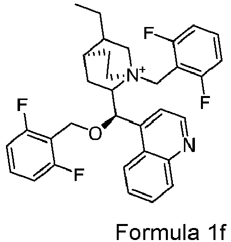 |
| HRMS ESI (m/z): | HRMS ESI (m/z): |
| calc. for C₃₃H3₃F₄N₂O [M]⁺: 549.2529, | calc. for C₃₃H₃₃F₄N₂O [M]⁺: 549.2529, |
| found: 549.2535. | found: 549.2527. |
| 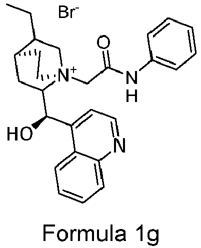 | 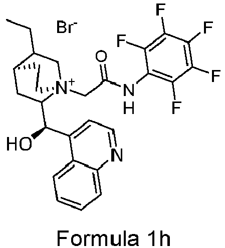 |
| HRMS ESI (m/z): | HRMS ESI (m/z): |
| calc. for C₂₇H₃₂N₃O₂ [M]⁺: 430.2495, | calc. for C₂₇H₂₇F₅N₃O₂ [M]⁺: 520.2023, |
| found: 430.2476. | found: 520.2001. |
| 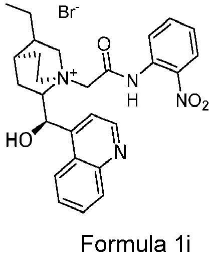 | 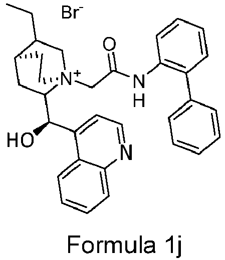 |
| HRMS ESI (m/z): | HRMS ESI (m/z): |
| calc. for C₂₇H₃₁ N₄O₄ [M]⁺: 475.2345, | calc. for C₃₃H₃₆N₃O₂ [M]⁺: 506.2808, |
| found: 475.2341. | found: 506.2814. |
| 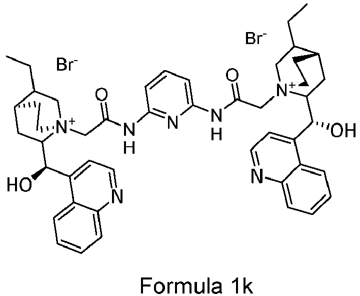 | |
| HRMS ESI (m/z): | |
| calc. for C₄₇H₅₇N₇O₄ [M]²⁺: 391.7231, | |
| found: 391.7241. | |

The subject matter of the invention in the following embodiments is shown in Examples 1-11, which illustrate the preparation of the compounds of the invention.

The subject matter of the invention in the following embodiments is shown in Tables 2-6 and in Examples 12-21, which show yield values and enantiomeric excesses for Schiff base alkylation reactions, enone epoxidation reactions, Michael addition (cyanation reaction) carried out in the presence of compounds of the invention as catalysts.

### Example 1. Synthesis of a compound of Formula 1a: N-(2-fluorobenzyl)-O-(2-fluorobenzyl) hydrocinchonidine bromide

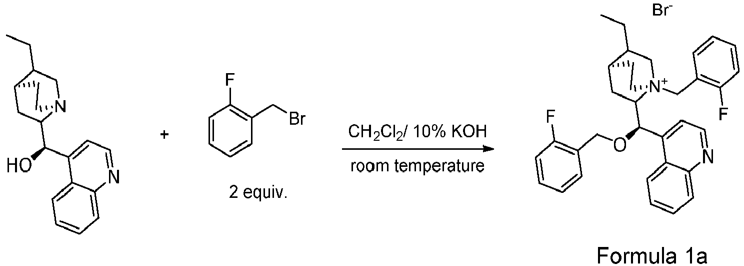

To a solution of hydrocinchonidine (200 mg, 0.67 mmol) and 2-fluorobenzyl bromide (165 µl, 1.37 mmol) in dichloromethane (3 mL), 10% KOH solution (2 mL) was added at room temperature. The reaction was carried out for 3 hours (under TLC control). The mixture was then diluted with water (2 mL) and the organic layer was extracted with dichloromethane (3 x 5 mL). The combined organic fractions were dried over anhydrous Na₂SO₄, filtered and evaporated under reduced pressure. The resulting residue was dissolved in dichloromethane (1 mL) and added dropwise slowly to diethyl ether (10 mL) at 0°C. The resulting precipitate was filtered off to give the product 374 mg (94 %) as a colorless solid (mp 133-134°C).
¹H NMR (400 MHz, DMSO) δ 9.04 (d, J = 4.4 Hz, 1H), 8.34 (d, J = 8.2 Hz, 1H), 8.16 (d, J = 8, 3 Hz, 1H), 7.89 (t, J = 7.5 Hz, 1H), 7.85 - 7.76 (m, 3H), 7.72 - 7.61 (m, 2H), 7 , 51 - 7.40 (m, 3H), 7.30 (t, J = 8.3 Hz, 2H), 6.64 (s, 1H), 5.28 (d, J = 12.5 Hz, 1H), 4.91 (dd, J = 16.9, 11.9 Hz, 2H), 4.57 (d, J = 11.0 Hz, 1H), 4.13 (t, J = 8.5 Hz, 1H), 3.98 (t, J = 10.4 Hz, 1H), 3.53 - 3.44 (m, 1H), 3.37 (d, J = 11.5 Hz, 1H), 3.18 (dd, J = 10.6, 5.7 Hz, 1H), 2.28 - 2.20 (m, 1H), 1.96 - 1.88 (m, 2H), 1.83 - 1.75 (m, 1H), 1.73 - 1.64 (m, 1H), 1.46 (t, J = 11.9 Hz, 1H), 1.29 - 1.12 (m, 2H), 0.69 (t, J = 7.3 Hz, 3H).
¹³C NMR (100 MHz, DMSO) δ 162.94, 161.8, 160.5, 150.2, 148.1, 140.8, 135.9, 133.3, 133.2, 131.1 (x2), 130.8, 130.7, 129.9, 129.6, 127.4, 125.1, 125.1, 124.8, 124.7, 123.8, 123.8, 123.6, 119.7, 116.4, 116.2, 115.6, 115.4, 115.2, 115.1, 67.5, 64.4, 61.4, 57.1, 50.9, 34, 9, 25.1, 24.7, 23.5, 20.6, 11.1.

### Example 2. Synthesis of compound of Formula 1b: N-(2-fluorobenzyl)-O-(2-fluorobenzyl) hydrocinchonine bromide

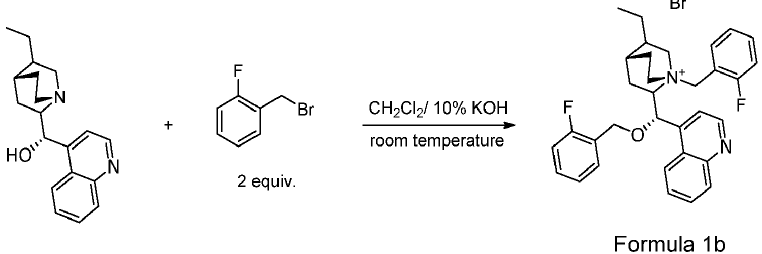

The synthesis of the compound of Formula **1b** was carried out as in Example 1. The product 378 mg (95 %) was obtained as a colorless solid (mp 134-135°C).
¹H NMR (400 MHz, DMSO) δ 9.06 (d, J = 4.4 Hz, 1H), 8.39 (d, J = 8.3 Hz, 1H), 8.16 (d, J = 8, 3 Hz, 1H), 7.89 (t, J = 7.6 Hz, 1H), 7.84 - 7.77 (m, 3H), 7.72 - 7.59 (m, 2H), 7 , 51 - 7.41 (m, 3H), 7.31 (dd, J = 15.6, 8.0 Hz, 2H), 6.60 (s, 1H), 5.04 (d, J = 12, 8 Hz, 1H), 4.91 (d, J = 12.7 Hz, 1H), 4.83 (d, J = 10.7 Hz, 1H), 4.59 (d, J = 10.7 Hz, 1H), 4.04 (t, J = 9.5 Hz, 1H), 3.96 (t, J = 10.9 Hz, 1H), 3.78 - 3.69 (m, 1H), 3.25 (d, J = 10.9 Hz, 1H), 3.07 (dd, J = 19.3, 10.4 Hz, 1H), 2.34 (t, J = 11.5 Hz, 1H), 1.84 - 1.66 (m, 4H), 1.37 - 1.26 (m, 2H), 1.23 - 1.14 (m, 1H), 0.64 (t, J = 7, 3 Hz, 3H).
¹³C NMR (100 MHz, DMSO) δ 150.2, 140.1, 135.7, 135.3, 133.3, 131.2, 131.2, 130.9, 130.8, 129.8, 129.6, 127.4, 125.1, 124.6, 123.9, 116.3, 115.6, 115.4, 115.2, 109.5, 67.0, 64.4, 56.4, 34.3, 24.2, 23.9, 23.3, 20.7, 10.8.

### Example 3. Synthesis of a compound of Formula 1c: N-(2-fluorobenzyl)-O-(2-fluorobenzyl) hydroquinine bromide

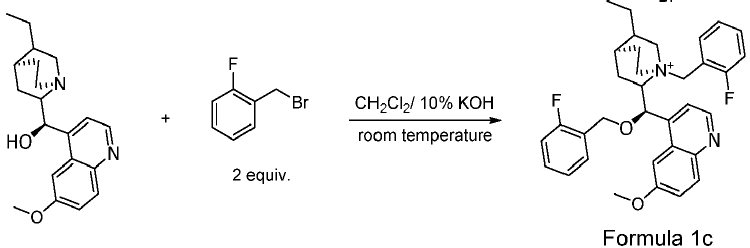

The synthesis of the compound of Formula **1c** was carried out as in Example 1. The product 372 mg (89 %) was obtained as a cream solid (mp 138-139°C).
¹H NMR (400 MHz, DMSO) δ 8.87 (d, J = 4.4 Hz, 1H), 8.06 (d, J = 9.2 Hz, 1H), 7.80 (t, J = 7 , 1 Hz, 1H), 7.74 - 7.65 (m, 3H), 7.55 - 7.42 (m, 5H), 7.30 (dd, J = 12.8, 5.0 Hz, 2H), 6.73 (s, 1H), 5.63 (d, J = 10.2 Hz, 1H), 5.01 (d, J = 10.9 Hz, 1H), 4.67 (d, J = 12.5 Hz, 1H), 4.56 (d, J = 10.9 Hz, 1H), 4.13 - 4.05 (m, 1H), 4.02 (s, 3H), 3, 95 (t, J = 11.0 Hz, 1H), 3.50 - 3.43 (m, 1H), 3.39 - 3.36 (m, 1H), 3.27 - 3.17 (m, 1H), 2.29 (d, J = 7.4Hz, 1H), 1.91 (d, J = 17.9Hz, 2H), 1.84-1.76 (m, 1H), 1.1 71 (t, J = 9.8 Hz, 1H), 1.53 (t, J = 12.0 Hz, 1H), 1.30 - 1.15 (m, 2H), 0.70 (t, J = 7.3 Hz, 3H).
¹³C NMR (100 MHz, DMSO) δ 161.7, 160.4, 159.3, 157.4, 147.5, 144.1, 139.4, 135.8, 133.3, 131.4, 131.0, 130.6, 125.2, 124.8, 124.7, 124.0, 123.8, 121.9, 116.5, 115.5, 115.3, 115.2, 115.1, 102.2, 67.9, 64.4, 61.1, 57.4, 55.4, 34.8, 25.0, 24.7, 23.6, 11.1.

### Example 4. Synthesis of a compound of Formula 1d: N-(2-fluorobenzyl)-O-(2-fluorobenzyl) hydroquinidine bromide

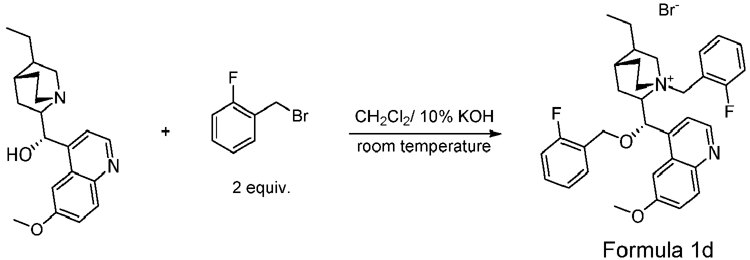

The synthesis of the compound of Formula **1d** was carried out as in Example 1. The product 380 mg (91%) was obtained as a cream solid (mp 137-138°C).
¹H NMR (400 MHz, DMSO) δ 8.88 (d, J = 4.4 Hz, 1H), 8.06 (d, J = 9.2 Hz, 1H), 7.76 (dd, J = 12 , 8, 5.9Hz, 2H), 7.73 - 7.63 (m, 2H), 7.56 - 7.42 (m, 5H), 7.32 (dd, J = 15.1.8 .0 Hz, 2H), 6.66 (s, 1H), 5.05 (d, J = 11.9 Hz, 1H), 4.89 (d, J = 10.6 Hz, 1H), 4. 73 (d, J = 9.3 Hz, 1H), 4.57 (d, J = 10.6 Hz, 1H), 4.07 (s, 3H), 3.99 (dd, J = 20.3 , 9.6 Hz, 2H), 3.79 - 3.69 (m, 1H), 3.39 - 3.33 (m, 1H), 3.03 (dd, J = 19.3, 10, 5 Hz, 1H), 2.47 - 2.38 (m, 1H), 1.86 - 1.65 (m, 4H), 1.37 - 1.28 (m, 2H), 1.26 - 1, 17 (m, 1H), 0.63 (t, J = 7.3Hz, 3H).
¹³C NMR (100 MHz, DMSO) δ 157.6, 147.5, 138.6, 135.6, 131.4, 131.2, 131.2, 125.3, 125.2, 124.6, 123.9, 121.8, 115.6, 115.3, 109.5, 102.3, 67.9, 64.4, 61.6, 56.2, 55.5, 34.4, 24.3, 24.0, 23.5, 10.8.

### Example 5. Synthesis of compound of Formula 1e: N-(2,4-difluorobenzyl)-O-(2,4-difluorobenzyl) hydrocinchonidine bromide

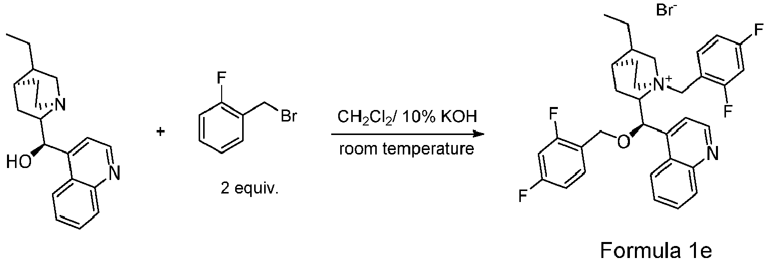

The synthesis of the compound of Formula **1e** was carried out as in Example 1, with a reaction time of 5 hours. The product 388 mg (92 %) was obtained as a colorless solid (mp 134-135 °C).
¹H NMR (400 MHz, DMSO) δ 9.04 (d, J = 4.2 Hz, 1H), 8.32 (d, J = 8.0 Hz, 1H), 8.15 (d, J = 8, 2 Hz, 1H), 7.95 - 7.86 (m, 2H), 7.83 - 7.66 (m, 3H), 7.53 (t, J = 8.6 Hz, 1H), 7 , 35 (dd, J = 20.4, 9.2 Hz, 2H), 7.17 (t, J = 7.3 Hz, 1H), 6.61 (s, 1H), 5.26 (d, J = 12.6 Hz, 1H), 4.89 (t, J = 12.2 Hz, 2H), 4.53 (d, J = 10.9 Hz, 1H), 4.13 - 4.04 (m, 1H), 3.92 (t, J = 10.1Hz, 1H), 3.46-3.38 (m, 2H), 3.19 (dd, J = 12.2, 8.9Hz, 1H), 2.23 (dd, J = 11.1, 6.5 Hz, 1H), 1.97 - 1.88 (m, 2H), 1.78 (d, J = 5.1 Hz, 1H), 1.69 (d, J = 8.4 Hz, 1H), 1.46 (t, J = 10.9 Hz, 1H), 1.26 - 1.12 (m, 2H), 0, 69 (t, J = 7.1 Hz, 3H).
¹³C NMR (100 MHz, DMSO) δ 150.1, 148.0, 140.8, 137.3, 132.5, 129.9, 129.7, 127.5, 125.1, 123.7, 119.8, 112.4, 111.9, 111.7, 104.9, 104.6, 104.4, 104.1, 103.8, 67.5, 63.8, 61.2, 56.5, 50.7, 35.0, 25.1, 24.7, 23.5, 11.1.

### Example 6. Synthesis of the compound of Formula 1f: N-(2,6-difluorobenzyl)-O-(2,6-difluorobenzyl) hydrocinchonidine bromide

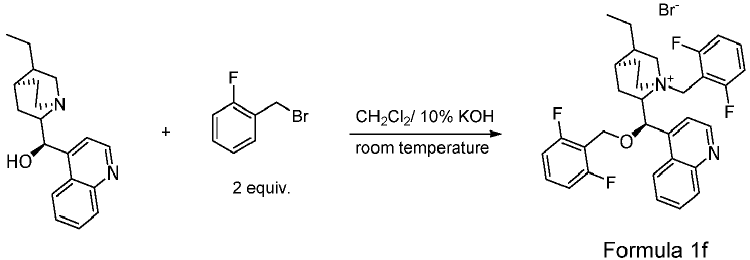

The synthesis of the compound of Formula **1f** was carried out as in Example 1, with a reaction time of 5 hours. The product 380 mg (90 %) was obtained as a colorless solid (mp 134-135 °C).
¹H NMR (400 MHz, DMSO) δ 9.05 (d, J = 4.4 Hz, 1H), 8.31 (d, J = 8.1 Hz, 1H), 8.16 (d, J = 8, 3 Hz, 1H), 7.89 (t, J = 7.5 Hz, 1H), 7.83 - 7.73 (m, 3H), 7.61 - 7.50 (m, 1H), 7 , 41 (t, J = 8.7 Hz, 2H), 7.22 (t, J = 7.9 Hz, 2H), 6.68 (s, 1H), 5.35 (d, J = 13, 0 Hz, 1H), 4.86 (d, J = 10.5 Hz, 1H), 4.71 (d, J = 13.1 Hz, 1H), 4.58 (d, J = 10.6 Hz, 1H), 4.31 - 4.23 (m, 1H), 3.92 (t, J = 9.5 Hz, 1H), 3.45 (t, J = 11.1 Hz, 1H), 3 , 30 - 3.20 (m, 2H), 2.20 (dd, J = 11.0, 4.2 Hz, 1H), 1.90 (s, 1H), 1.81 (d, J = 8, 1 Hz, 2H), 1.68 (t, J = 9.9 Hz, 1H), 1.47 (t, J = 11.9 Hz, 1H), 1.30 - 1.13 (m, 2H), 0.68 (t, J = 7.3 Hz, 3H).
¹³C NMR (100 MHz, DMSO) δ 160.0, 150.0, 148.1, 140.6, 134.4, 131.9, 129.8, 129.7, 127.3, 125.2, 124.0, 119.7, 114.1, 112.7, 112.5, 112.1, 112.0, 111.7, 104.8, 67.7, 64.9, 57.7, 52.3, 50.6, 34.9, 24.8, 24.8, 23.3, 20.3, 11.1.

### Example 7. Two-step synthesis of a compound of Formula 1g: N-(N'-phenylacetamido) hydrocynchonidine bromide

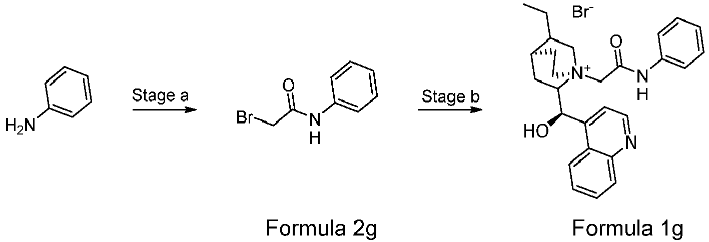

### Stage a

Aniline (1.0 g, 10.7 mmol) was dissolved in dichloromethane (15 mL) and the solution of K₂CO₃ (2.23 g, 16.1 mmol) in water (20 mL) was added. The reaction mixture was cooled to 0 °C and a solution of bromoacetyl bromide (3.24 g, 16.1 mmol, 1.4 mL) in dichloromethane (3.6 mL) was slowly added dropwise. The reaction was carried out under TLC control for approximately 1 hour, then the phases were separated and the aqueous was extracted with dichloromethane (3 x 20 mL). The combined organic phases were dried over anhydrous Na₂SO₄ and the solvent was evaporated under reduced pressure to give the product 2.1 g (92 %) as a colorless solid (mp 132-133°C).
¹H NMR (400 MHz, DMSO) δ 10.35 (s, 1H), 7.59 (d, J = 7.9 Hz, 2H), 7.33 (t, J = 7.9 Hz, 2H), 7.08 (t, J = 7.4 Hz, 1H), 4.04 (s, 2H).
¹³C NMR (100 MHz, DMSO) δ 164.7, 138.6, 128.8, 123.8, 119.2, 30.4.

### Stage b

To a solution of hydrocinchonidine (1.0 g, 3.4 mmol) in tetrahydrofuran (30 mL) amide **2g** (0.73 g, 3.4 mmol) was added. The mixture was refluxed for approximately 1.5 hours under TLC control. At this time, the solvent was evaporated under reduced pressure and the dry residue was dissolved in dichloromethane (2 mL). The thus prepared catalyst solution was slowly added dropwise to diethyl ether (15 mL). The resulting precipitate was filtered off, washed with diethyl ether and then dried to give the product 1.6 g (93 %) as a colorless solid (mp 153-154°C).
¹H NMR (400 MHz, DMSO) δ 11.07 (s, 1H), 8.97 (d, J = 4.4 Hz, 1H), 8.17 (d, J = 8.4 Hz, 1H), 8.07 (d, J = 8.3 Hz, 1H), 7.82 - 7.75 (m, 4H), 7.51 (t, J = 7.6 Hz, 1H), 7.44 (t, J = 7.8 Hz, 2H), 7.20 (t, J = 7.4 Hz, 1H), 6.71 (d, J = 3.7 Hz, 1H), 6.18 (bs, 1H), 4.88 (d, J = 15.9 Hz, 1H), 4.72 (d, J = 15.8 Hz, 1H), 4.40 (t, J = 11.4 Hz, 1H), 4.26 (t, J = 9.0 Hz, 1H), 3.98 (t, J = 11.3 Hz, 1H), 3.89 (d, J = 11.5 Hz, 1H), 3, 75 (dd, J = 11.3, 7.0 Hz, 1H), 2.16 - 1.86 (m, 5H), 1.34 - 1.13 (m, 3H), 0.72 (t, J = 7.3Hz, 3H).
¹³C NMR (100 MHz, DMSO) δ 162.5, 150.1, 147.6, 144.9, 137.7, 129.9, 129.3, 129.0, 127.0, 124.6, 124.3, 123.0, 120.2, 119.7, 65.1, 64.9, 63.8, 59.5, 55.5, 35.5, 25.7, 25.4, 23.4, 20.2, 11.3.

### Example 8. Two-step synthesis of the compound of Formula 1h: N-(N'-perfluoro-phenylacetamido) hydrocinchonidine bromide

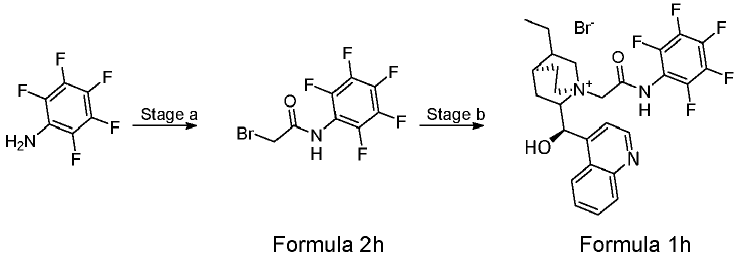

The two-step synthesis of the compound of Formula **1h** was carried out as in Example 7. Amide **2h** 3.1 g (94 %) was obtained as a colorless solid (mp 201-202°C).
¹H NMR (400 MHz, DMSO) δ 10.52 (s, 1H), 4.14 (s, 2H).
¹³C NMR (100 MHz, DMSO) δ 165.5, 28.2.

Compound of formula **1h** 1.9 g (94 %) was obtained as a colorless solid (mp 188-190°C).
¹H NMR (400 MHz, DMSO) δ 11.30 (s, 1H), 8.98 (d, J = 4.4 Hz, 1H), 8.10 (t, J = 8.9 Hz, 2H), 7.84 - 7.76 (m, 2H), 7.58 (t, J = 7.4 Hz, 1H), 6.80 (d, J = 3.4 Hz, 1H), 6.13 (bs, 1H), 4.98 (d, J = 16.2 Hz, 1H), 4.89 (d, J = 16.4 Hz, 1H), 4.43 (t, J = 10.9 Hz, 1H), 4.27 (t, J = 8.2 Hz, 1H), 3.98 (t, J = 11.2 Hz, 1H), 3.79 - 3.70 (m, 2H), 2.15 - 1.85 (m, 5H), 1.30 - 1.11 (m, 3H), 0.71 (t, J = 7.2 Hz, 3H).
¹³C NMR (100 MHz, DMSO) δ 163.7, 150.2, 147.6, 144.7, 129.9, 129.5, 127.0, 124.3, 123.0, 120.2, 65.4, 65.0, 63.4, 58.6, 55.5, 35.5, 25.7, 25.3, 23.4, 20.4, 11.2.

### Example 9. Two-step synthesis of the compound of Formula 1i: N-(N-2-nitro-acetamido)hydrocynchonidine bromide

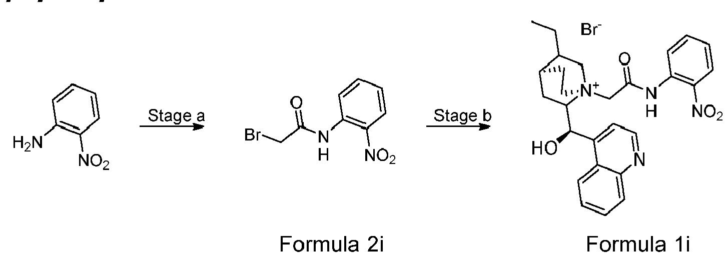

The two-step synthesis of the compound of Formula **1i** was carried out as in Example 7. Amide **2i** 2.7 g (97 %) was obtained as a cream solid (mp 62-63°C).
¹H NMR (400 MHz, DMSO) δ 10.67 (s, 1H), 8.00 (dd, J = 8.2, 0.8 Hz, 1H), 7.76 - 7.69 (m, 2H), 7.41 (ddd, J = 8.5, 6.5, 2.3 Hz, 1H), 4.14 (s, 2H).
¹³C NMR (100 MHz, DMSO) δ 165.1, 142.2, 134.1, 130.6, 125.8, 125.2, 125.0, 29.4. Compound of formula **1i** 1.7 g (92 %) was obtained as a cream solid (mp 146-148°C).
¹H NMR (400 MHz, DMSO) 11.38 (s, 1H), 8.98 (d, J = 4.4 Hz, 1H), 8.16 (d, J = 8.4 Hz, 1H), 8. 08 (d, J = 8.3 Hz, 2H), 7.85 (t, J = 7.3 Hz, 1H), 7.82 - 7.76 (m, 2H), 7.70 (d, J = 7.8 Hz, 1H), 7.56 (t, J = 5.0 Hz, 2H), 6.79 (d, J = 3.7 Hz, 1H), 6.18 (bs, 1H), 4.88 (d, J = 15.9 Hz, 1H), 4.70 (d, J = 15.9 Hz, 1H), 4.42 (t, J = 12.5 Hz, 1H), 4 , 24 (t, J = 9.0 Hz, 1H), 3.95 (t, J = 11.2 Hz, 1H), 3.75 - 3.62 (m, 2H), 2.13 - 1,88 (m, 5H), 1.27-1.08 (m, 3H), 0.72 (t, J = 7.3 Hz, 3H).
¹³C NMR (100 MHz, DMSO) δ 163.2, 150.1, 149.5, 147.6, 144.9, 143.4, 134.3, 129.8, 129.4, 129.1, 127.0, 126.3, 125.2, 124.4, 123.2, 120.1, 65.4, 65.1, 63.5, 59.3, 55.6, 35.5, 25.7, 23.4, 20.4, 19.8, 11.2.

### Example 10. Two-step synthesis of the compound of Formula 1j: N-(N'-2-phenyl-phenylacetamido) hydrocinchonidine bromide

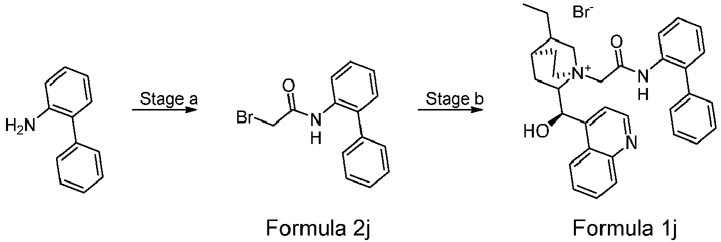

The two-step synthesis of the compound of Formula **1j** was carried out as in Example 7. Amide **2j** 2.9g (92 %) was obtained as a colorless solid (mp 86-87°C).
¹H NMR (400 MHz, DMSO) δ 9.64 (s, 1H), 7.54 (d, J = 7.7 Hz, 1H), 7.48 - 7.43 (m, 2H), 7.41 - 7.30 (m, 6H), 3.95 (s, 2H).
¹³C NMR (100 MHz, DMSO) δ 165.1, 138.3, 136.3, 134.0, 130.3, 128.8, 128.4, 127.8, 127.4, 126.5, 126.3, 29.6.

The compound of Formula **1j** 1.8 g (92 %) was obtained as a colorless solid (mp 152-153°C).
¹H NMR (400 MHz, DMSO) δ 10.44 (s, 1H), 8.97 (d, J = 4.4 Hz, 1H), 8.10 (dd, J = 19.6, 8.4 Hz , 2H), 7.82 - 7.74 (m, 2H), 7.60 - 7.54 (m, 2H), 7.49 - 7.35 (m, 7H), 7.27 (t, J = 7.3 Hz, 1H), 6.67 (d, J = 3.7 Hz, 1H), 6.00 (bs, 1H), 4.67 (d, J = 15.6 Hz, 1H), 4.46 (d, J = 15.6 Hz, 1H), 4.29 (t, J = 12.4 Hz, 1H), 4.19 (t, J = 8.7 Hz, 1H), 3, 70 - 3.55 (m, 2H), 3.44 (d, J = 12.2 Hz, 1H), 2., 10 - 1.79 (m, 5H), 1.20 - 1.04 (m , 3H), 0.69 (t, J = 7.3 Hz, 3H).
¹³C NMR (100 MHz, DMSO) δ 162.9, 150.1, 147.6, 145.0, 138.7, 137.8, 133.0, 130.6, 129.8, 129.4, 128.7, 128.5, 128.1, 127.4, 127.2, 127.1, 127.0, 124.3, 123.3, 120.0, 65.3, 65.1, 63.2, 59.2, 55.1, 35.5, 25.6, 25.3, 23.2, 16.0, 11.3.

### Example 11. Two-step synthesis of a compound of Formula 1k: N,N'-(pyridin-2,6-diyl)bis(2-hydrocinchonidinacetamide) dibromide

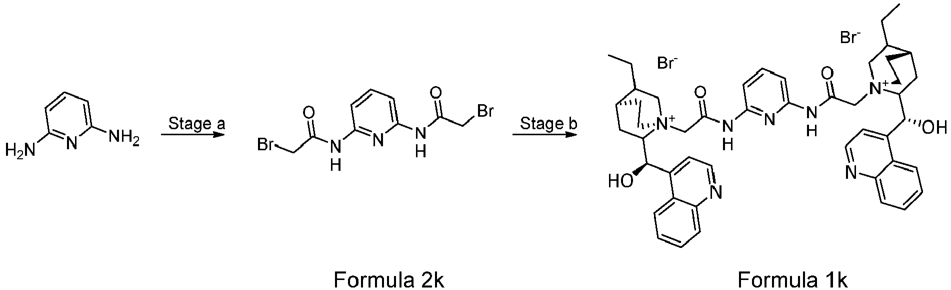

### Stage a

2,6-diaminopyridine (1.0 g, 9.2 mmol) was dissolved in dichloromethane (20 mL) and solution of K₂CO₃ (3.83 g, 27.7 mmol) in water (25 mL) was added. The reaction mixture was cooled to 0 °C and a solution of bromoacetyl bromide (5.6 g, 27.7 mmol, 2.4 mL) in dichloromethane (2.6 mL) was slowly added dropwise. The reaction was carried out under TLC control for approximately 1 hour, then the resulting precipitate was filtered off, washed with distilled water and diethyl ether, and then dried under reduced pressure. The product, 2.87 g (89 %), was obtained as a cream solid (mp 140-141 °C (decomposition)).
¹H NMR (400 MHz, DMSO) δ 10.50 (s, 2H), 7.85-7.69 (m, 3H), 4.17 (s, 4H).
¹³C NMR (100 MHz, DMSO) δ 165.7, 149.8, 140.5, 109.6, 30.2.

### Stage b

To a solution of hydrocinchonidine (1.0 g, 3.4 mmol) in tetrahydrofuran (50 mL) diamide **2k** was added (0.60 g, 1.7 mmol). The mixture was refluxed for approximately 3 hours under TLC control. After this time, the solvent was evaporated under reduced pressure, suspended in acetone/diethyl ether [1:1, v/v] and sonicated for 5 minutes. The resulting precipitate was filtered off, washed with diethyl ether and dried under reduced pressure. The product, 1.4 g (87 %), was obtained as a light orange solid (mp 205-206°C (decomposition)).
¹H NMR (400 MHz, DMSO) δ 11.11 (s, 2H), 8.98 (d, J = 4.4 Hz, 2H), 8.18 - 8.01 (m, 7H), 7.84 - 7.74 (m, 5H), 7.57 (t, J = 7.2 Hz, 2H), 6.72 (s, 2H), 6.17 (s, 2H), 4.99 (d, J = 15.7 Hz, 2H), 4.83 (d, J = 15.6 Hz, 2H), 4.40 (t, J = 10.9 Hz, 2H), 4.25 (t, J = 8.3 Hz, 2H), 4.01 (t, J = 11.1 Hz, 2H), 3.87 - 3.73 (m, 3H), 2.18 - 1.86 (m, 10H), 1.35 - 1.13 (m, 6H), 0.72 (t, J = 7.2 Hz, 6H).
¹³C NMR (100 MHz, DMSO) δ 163.9, 150.2, 149.3, 149.2, 147.6, 145.9, 144.9, 130.0, 129.4, 127.0, 124.3, 122.9, 120.3, 65.3, 64.9, 62.7, 59.5, 55.4, 35.5, 25.7, 25.4, 23.4, 20.2, 11.3.

### Example 12. The use of compounds of the invention of Formula 1a-f as catalysts for asymmetric alkylation of Schiff bases with imine derivatives of α-amino acid esters as substrates.

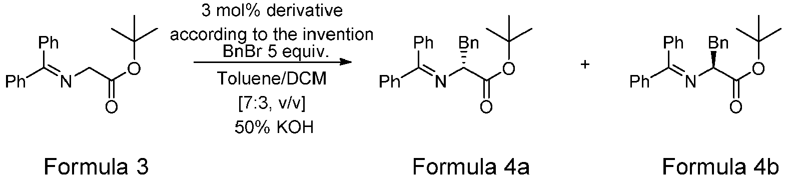

The compound of the invention (0.01 mmol) and *N*-(diphenylmethylene)-glycine *tert*-butyl ester 3 (59 mg, 0.2 mmol) were dissolved in a toluene/chloroform [7:3, v/v] mixture (1.0 mL). Then benzyl bromide (1.0 mmol, 120 µL) and 50% KOH solution (0.3 mL) were added. The reaction was carried out at 0 °C for 2-12 hours (TLC control). After the reaction was complete, the mixture was diluted with water (2 mL) and the organic layer was extracted with dichloromethane (3 x 2 mL). The combined organic fractions were dried over anhydrous Na₂SO₄, filtered and evaporated. The obtained residue was purified by column chromatography using hexane/ethyl acetate [99:1 → 95:5, v/v] as an eluent, to give the product as a colorless oil. The results are shown in Table 2.

Table 2. Summary of the results obtained for the asymmetric alkylation of Schiff bases with compound **3** as a substrate and the compound of the invention as a catalyst.

| Entry | Derivative according to the invention, Formula No. | The C₉ carbon configuration of the compound of Formula | Stereochemistry of the major enantiomer | Solvent | Temperature [°C] | ee [%] | Yield [%] |
|---|---|---|---|---|---|---|---|
| 1 | 1a | *R* | *S* | CHCl₃/Toluene | -20 | 96 | 97 |
| 2 | 1a | *R* | *S* | CH₂Cl₂/Toluene | -20 | 95 | 95 |
| 3 | 1b | *S* | *R* | CHCl₃/Toluene | -20 | 91 | 90 |
| 4 | 1c | *R* | *S* | CHCl₃/Toluene | -20 | 83 | 92 |
| 5 | 1d | *S* | *R* | CHCl₃/Toluene | -20 | 63 | 91 |
| 6 | 1a | *R* | *S* | CH₂Cl₂/Toluene | -10 | 94 | 96 |
| 7 | 1a | *R* | *S* | CHCl₃/Toluene | -40 | 98 | 95 |

| | | | | | | | |
|---|---|---|---|---|---|---|---|
| The enantiomeric excess was determined by chiral high performance liquid chromatography (HPLC), using a Daicel Chiralcel OD-H column, using hexane/isopropanol [99.3:0.7, v/v] as eluent, flow = 0.5 mL/min, with detection (UV-DAD) at λ = 254 nm. Retention times: 17.6 min (R) and 28.6 min (S). | | | | | | | |

### Example 13. The use of compound of the invention of Formula 1a as a catalyst for an asymmetric alkylation reaction involving allyl bromide.

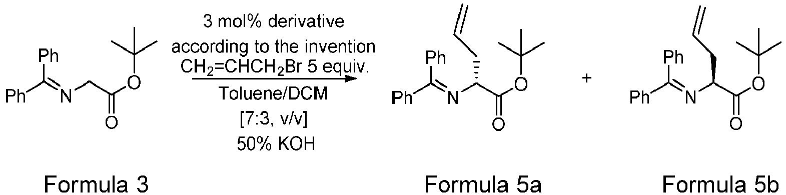

For the compound of the invention of Formula **1a,** the reaction was carried out according to the procedure of Example 12, using allyl bromide as the alkylating agent. The results of the enantiomeric excess and the yield are shown in Table 3.

**Table 3. Summary of the results obtained for the asymmetric alkylation of Schiff bases with compound 3 as substrate, allyl bromide and the compound of the invention of Formula 1a as catalyst.**

| Entry | Derivative according to the invention, Formula No. | The C₉ carbon configuration of the compound of Formula | Stereochemistry of the major enantiomer | Solvent | Temperature [°C] | ee [%] | Yield [%] |
|---|---|---|---|---|---|---|---|
| 1 | 1a | *R* | *S* | CHCl₃/Toluene | -20 | 96 | 97 |
| 2 | 1a^{a} | *R* | *S* | CH₂Cl₂/Toluene | -15 | 94 | 96 |
| 3 | 1a^{b} | *R* | *S* | CH₂Cl₂/Toluene | -15 | 94 | 98 |

| | | | | | | | |
|---|---|---|---|---|---|---|---|
| The enantiomeric excess was determined by chiral high performance liquid chromatography (HPLC), using a Daicel Chiralcel OD-H column, using hexane/isopropanol [99.3:0.7, v/v] as eluent, flow = 0.5 mL/ min, with detection (UV-DAD) at λ = 254 nm. Retention times: 10.1 min (R) and 11.1 min (S). ^{a}Reaction conducted using an *in situ* generated catalyst. ^{b}Reaction conducted under flow conditions with simultaneous generation of the catalyst *in situ.* | | | | | | | |

### Example 14. Synthesis of the compound of the invention of Formula 1a and subsequent asymmetric alkylation reaction with allyl bromide.

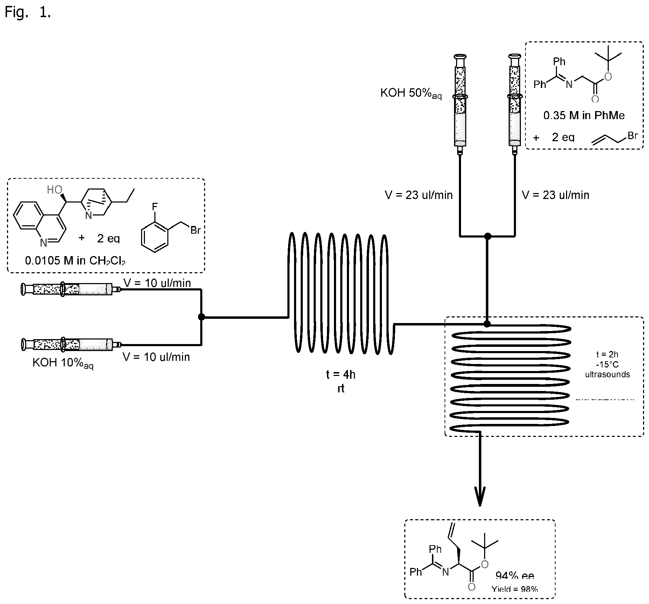

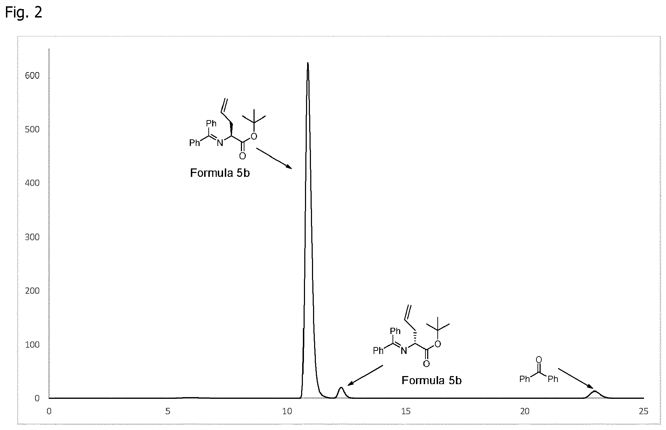

The synthesis of the catalyst of Formula **1a** was carried out by flow technique by mixing a 0.0105 M solution of hydrocinchonidine and 2 molar equivalents of 2-fluorobenzyl bromide in dichloromethane with a 10% aqueous KOH solution. The reaction was carried out with the flow of the organic and aqueous phases of 10 µL/min each. The volume of the reactor was adjusted to allow the reaction to run for 4 hours at room temperature. Then, a mixture of 0.35 M solution of compound **3** as substrate and 5 molar equivalents of allyl bromide in toluene, as well as 50% aqueous KOH solution, was added to the catalyst thus generated *in situ.* The flow rate of both the substrate and the KOH solution was set to 23 µL/min each, keeping the catalyst **1a** concentration constant at 3 mol%. The reactor was placed in an ultrasonic bath and its volume was adjusted to allow the reaction to run for 2 hours at -15 °C. The system is shown in Fig. 1. HPLC analysis was performed to determine the yield and the enantiomeric excess. Fig. 2 shows the resulting chromatogram. The results are shown in Table 3.

### Example 15. One-pot synthesis of a compound of the invention of Formula 1a and subsequent asymmetric alkylation reaction with allyl bromide.

To a solution of hydrocinchonidine (0.015 g, 0.05 mmol) in dichloromethane (8 mL) 2 molar equivalents of 2-fluorobenzyl bromide (12 µL, 0.1 mmol) and a 10% KOH solution (5 mL) were added. The system thus obtained was stirred at room temperature for 4 hours. At this time, to a crude mixture of compound **3** (0.5 g, 1.7 mmol) in toluene (18.5 mL), 5 molar equivalents of allyl bromide (0.74 mL, 8.5 mmol), as well as 50% KOH solution (5.8 ml) were added. The reaction was carried out at -15 °C for 3 hours. After the reaction was complete, the mixture was diluted with water (10 mL) and the organic layer was extracted with dichloromethane (3 x 10 mL). The combined organic fractions were dried over anhydrous Na₂SO₄, filtered and evaporated. The obtained residue was purified by column chromatography, using hexane/ethyl acetate [99:1 → 95:5, v/v] as an eluent, to give the product as a colorless oil. The results are shown in Table 3.

### Example 16. Use of a compound of the invention of Formula 1a as a catalyst for an asymmetric alkylation reaction involving substituted benzyl bromides.

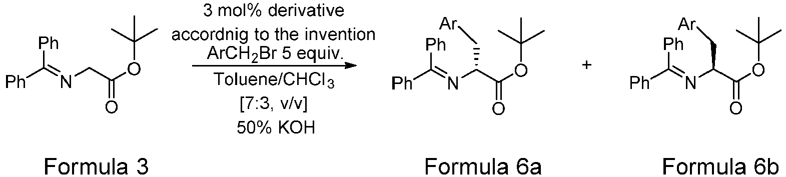

**Table 4. Summary of the results obtained for the asymmetric alkylation of Schiff bases with compound 3 as substrate, substituted benzyl bromides and the compound of the invention of Formula 1a as catalyst.**

| Entry | Alkylating agent | Major enantiomer | ee [%] | Yield [%] | Retention time [min] | |
|---|---|---|---|---|---|---|
| | | | | | *R* (Formula **6a**) | *S* (Formula **6b**) |
| 1 | PhCH₂Br | *S* | 96 | 97 | 17.6 | 28.6 |
| 2 | 2-F-PhCH₂Br | *S* | 96 | 98 | 14.7 | 15.8 |
| 3 | 3-F-PhCH₂Br | *S* | 96 | 97 | 15.2 | 19.2 |
| 4 | 4-F-PhCH₂Br | *S* | 97 | 98 | 12.6 | 17.6 |
| 5 | 2-Me-PhCH₂Br | *S* | 97 | 98 | 20.2 | 31.8 |
| 6 | 3-Me-PhCH₂Br | *S* | 97 | 97 | 17.3 | 22.2 |
| 7 | 4-Me-PhCH₂Br | *S* | 97 | 98 | 12.7 | 14.4 |
| 8 | 4-*t*-Bu-PhCH₂Br | *S* | 97 | 99 | 10.3 | 12.0 |

| | | | | | | |
|---|---|---|---|---|---|---|
| The enantiomeric excess was determined by chiral high performance liquid chromatography (HPLC), using a Daicel Chiralcel OD-H column, using hexane/isopropanol [99.3:0.7, v/v] as eluent, flow = 0.5 mL/min, with detection (UV-DAD) at A = 254 nm. | | | | | | |

The hydrocinchonidine derivatives and analogs of the invention have also been used in other exemplary PTC reactions performed under conditions known in the art. The results obtained are presented in the following Examples 17-19.

### Example 17. Use of a compound of the invention of Formula 1a as a catalyst in the asymmetric Michael addition reaction to N-(diphenylmethylene) glycine tert-butyl ester

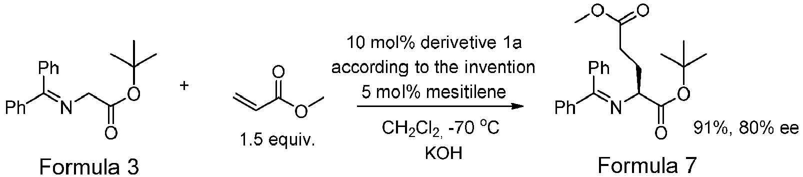

The compound of the invention of Formula **1a** (8.7 mg, 0.02 mmol) and mesitilene (1.15 mg, 5 mol%) were dissolved in dichloromethane (0.5 mL) at 0 °C and KOH (19 mg, 0.34 mmol) was added. Stirring was continued for 30 min. The mixture was then cooled to -70 °C and a solution of *N*-(diphenylmethylene)-glycine *tert*-butyl ester **3** (50 mg, 0.01 mmol) and methyl acrylate (23 µL, 0.26 mmol) in dichloromethane (0.5 mL) was added. The reaction was carried out at this temperature for 2 hours (TLC control). After the reaction was complete, the solvents were evaporated under reduced pressure and the dry residue was purified by column chromatography, using hexane/ethyl acetate [9:1, v/v] as an eluent to give the product **7** (59 mg, 0.18 mmol) as a colorless oil (80 % ee, Y = 91 %).

The enantiomeric excess was determined by chiral high performance liquid chromatography (HPLC) using conditions according to Lygo, B., Beynon, C., Lumley, C., McLeod, M.C., Wade, C.E., Tetrahedron Letters, 2009, 50, 3363.

### Example 18: Use of a compound of the invention of Formula 1b as a catalyst in an asymmetric epoxidation of trans-chalcone

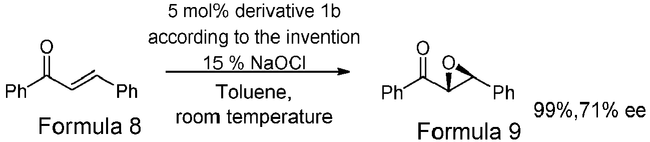

To a solution of the enone of Formula **8** (20.8 mg, 0.1 mmol) and the compound of the invention **1b** (4.3 mg, 0.005 mmol) in toluene (0.5 mL) 15 % NaOCI (0.8 mL) was added. The resulting two-phase system was vigorously mixed (1000 rpm). The reaction was carried out at room temperature for 7 hours (TLC control). After the reaction was complete, the mixture was diluted with water (2 mL) and the organic layer was extracted with dichloromethane (3 x 2 mL). The combined organic fractions were dried over anhydrous Na₂SO₄, filtered and evaporated under reduced pressure. The obtained residue was purified by chromatography in the elution system with hexane/ethyl acetate [9:1, v/v] to give the product **9** as a colorless solid (71% ee, Y = 99 %).

The enantiomeric excess was determined by chiral high performance liquid chromatography (HPLC) using literature conditions: [Lygo, B., Gardiner, S.T., McLeod, M.C., To, D.C, Org. Biomol. Chem., 2007, 5, 2283]

### Example 19. Use of a compound of the invention of Formula 1a as a catalyst in an asymmetric cyanation reaction of β-phenyl-β-trifluoromethyl enone

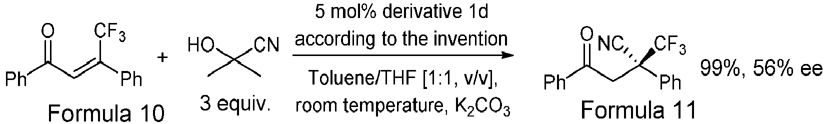

To a solution of the enone **10** (28 mg, 0.1 mmol) and the compound of the invention **1d** (3.1 mg, 0.005 mmol) in a THF/toluene mixture [1:1, v/v] K₂CO₃ (42 mg, 0.3 mmol) and acetone cyanohydrin (28 µL, 0.3 mmol) were added. The reaction was carried out at room temperature for 8 hours (TLC control). After the reaction was complete, the solvents were evaporated under reduced pressure and the dry residue was purified by column chromatography using hexane/ethyl acetate [9:1, v/v] as an eluent to give the product **11** (30 mg, 0.099 mmol) as a colorless solid (56 % ee , Y = 99 %).

The enantiomeric excess was determined by chiral high performance liquid chromatography (HPLC) using the conditions according to Kawai, H., Okusu, S., Takunaga, E., Sato, H., Shiro, M., Shibata, N., Angew. Chem. Int. Ed., 2012, 51, 4959.

### Example 20. Use of compounds of the invention of Formulas 1g-1k as catalysts for asymmetric alkylation of Schiff bases with imine derivatives of α-amino acid esters as substrates.

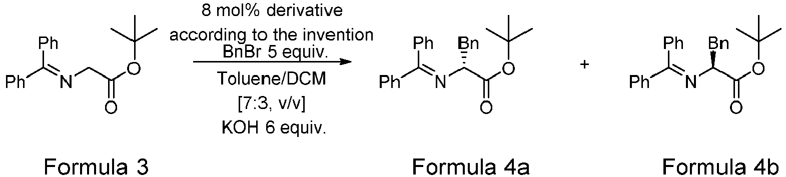

The compound of the invention (0.016 mmol) and *N*-(diphenylmethylene)-glycine *tert*-butyl ester **3** (59mg, 0.2 mmol) were dissolved in a toluene/chloroform [7:3, v/v] mixture (0.8 mL). Then benzyl bromide (1.0 mmol, 120 µL) and 50 % KOH solution (0.2 mL) were added. The reaction was carried out at -20 °C for 6-12 hours (TLC control). After completion of the reaction, the solvents were evaporated under reduced pressure, and the dry residue was purified by column chromatography using hexane/ethyl acetate [99:1 → 95: 5, v/v] as an eluent, to give the product as a colorless oil. The results are shown in Table 5.

**Table 5. Summary of the results obtained for the asymmetric alkylation of Schiff bases with compound 3 as a substrate and the corresponding compound of the invention as a catalyst.**

| Entry | Derivative according to the invention, Formula No. | The C₉ carbon configuration of the compound of Formula | Stereochemistry of the major enantiomer | ee [%] | Yield [%] |
|---|---|---|---|---|---|
| 1 | 1g | *R* | *S* | 62 | 89 |
| 2 | 1h | *R* | *S* | 91 | 90 |
| 3 | 1h*^{a}* | *R* | *S* | 80 | 95 |
| 4 | 1i | *R* | *S* | 92 | 90 |
| 5 | 1j | *R* | *S* | 60 | 88 |
| 6 | 1k | *R* | *S* | 90 | 93 |

| | | | | | |
|---|---|---|---|---|---|
| The enantiomeric excess was determined by chiral high performance liquid chromatography (HPLC), using a Daicel Chiralcel OD-H column, using hexane/isopropanol [99.3:0.7, v/v] as eluent, flow = 0.5 mL/min, with detection (UV-DAD) at λ = 254 nm. Retention times: 17.6 min (R) and 28.6 min (S). ^{a} Reaction carried out at room temperature. | | | | | |

### Example 21. Use of compound of the invention of Formula 1h as a catalyst for an asymmetric alkylation reaction involving substituted benzyl bromides.

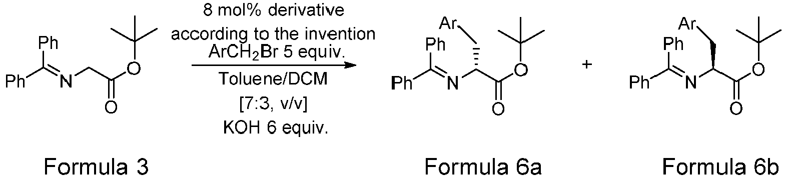

For the compound of the invention of Formula **1h,** the reaction was carried out according to the procedure of Example 20, using a series of substituted benzyl bromides as alkylating agents. The results of the enantiomeric excess and the yield are shown in Table 6.

**Table 6. Summary of the results obtained for the asymmetric alkylation of Schiff bases with compound 3 as a substrate, a series of alkylating agents and the compound of the invention of Formula 1h as catalyst.**

| Entry | Alkylating agent | Major enantiomer | ee [%] | Yield [%] | Retention time [min] | |
|---|---|---|---|---|---|---|
| | | | | | *R* (Formula **6a**) | *S* (Formula **6b**) |
| 1 | BnBr | *S* | 91 | 90 | 17.6 | 28.6 |
| 2 | 2-F-PhCH₂Br | *S* | 91 | 92 | 12.0 | 13.0 |
| 3 | 3-F-PhCH₂Br | *S* | 83 | 90 | 15.5 | 19.7 |
| 4 | 4-F-PhCH₂Br | *S* | 86 | 94 | 13.4 | 18.9 |
| 5 | 2-Cl-PhCH₂Br | *S* | 91 | 91 | 19.0 | 20.6 |
| 6 | 4-Me-PhCH₂Br | *S* | 87 | 95 | 14.2 | 16.0 |
| 7 | 4-*t*-Bu-PhCH₂Br | *S* | 81 | 96 | 11.1 | 13.1 |

| | | | | | | |
|---|---|---|---|---|---|---|
| The enantiomeric excess was determined by chiral high performance liquid chromatography (HPLC), using a Daicel Chiralcel OD-H column, using hexane/isopropanol [99.3:0.7, v/v] as eluent, flow = 0.5 mL/min, with detection (UV-DAD) at A = 254 nm. | | | | | | |

On the basis of the obtained results, it was found that the newly developed and newly prepared compounds according to the invention can be used as effective catalysts in asymmetric reactions occurring under the PTC conditions (Table 2-6 and Examples 17-19). The compounds of the invention find use as PTC catalysts under both liquid-liquid and liquid-solid conditions, as has been demonstrated in other reactions (Examples 17-19).

In particular, the added value of *N,O*-disubstituted derivatives is the fact that they can be generated *in situ* in the flow conditions without separating them from the reaction mixture.

## Claims

1. Compound of general Formula 1: 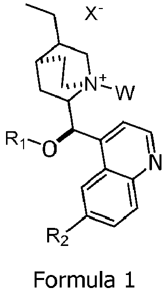 wherein:
**R₁** is independently hydrogen, C₁-C₅ alkyl, C₅-C₁₆ aryl, or substituted benzyl of formula **A,** wherein A is: 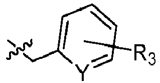 and **R₃** is independently hydrogen, halogen, trifluoromethyl (-CF₃), nitrile (-CN) or nitro (-NO₂) group and **Y** is any heteroatom or CH group;
**R₂** is independently hydrogen or methoxy group (-OCH₃);
**X⁻** is any inorganic anion;
**W** is said **A,** wherein **Y** and **R₃** are as above; or **W** is 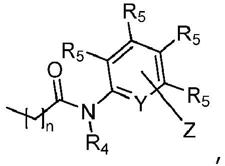 wherein **n** is an integer ranging from 1 to 5; **R₄** is independently hydrogen, C₁-C₅ alkyl, or C₅-C₁₆ aryl; **R₅** is independently hydrogen, halogen, C₁-C₁₂ alkyl, C₅-C₁₆ aryl, C₁-C₅ branched alkyl, trifluoromethyl (-CF₃), nitrile (-CN), nitro (-NO2), alkoxy -OC₁- C₁₂ group or **Z** of the formula 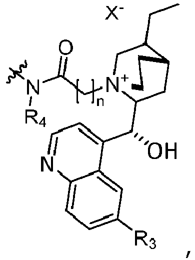
wherein **Y, n, R₃**, **R₄** and **X** are as above.

2. The compound according to claim 1, wherein the compound of Formula 1 is selected from:

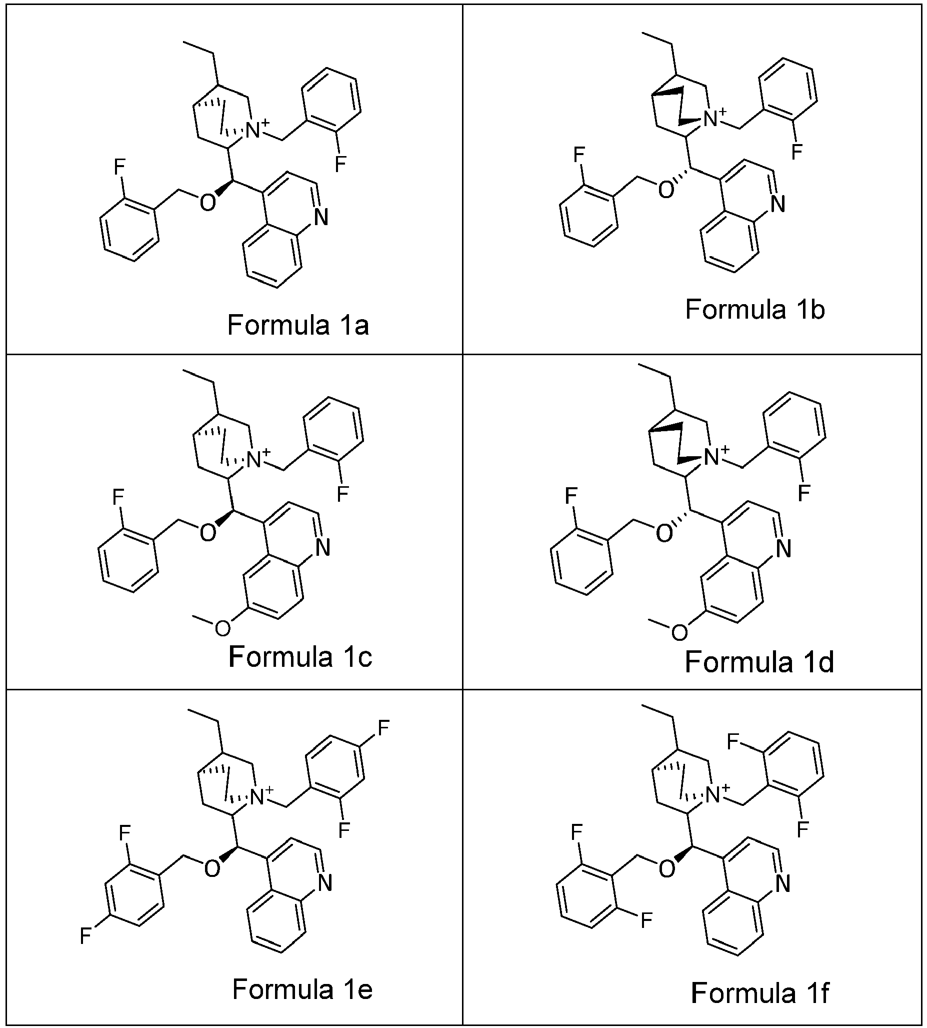

3. The compound according to claim 1, wherein the compound of Formula 1 is selected from:

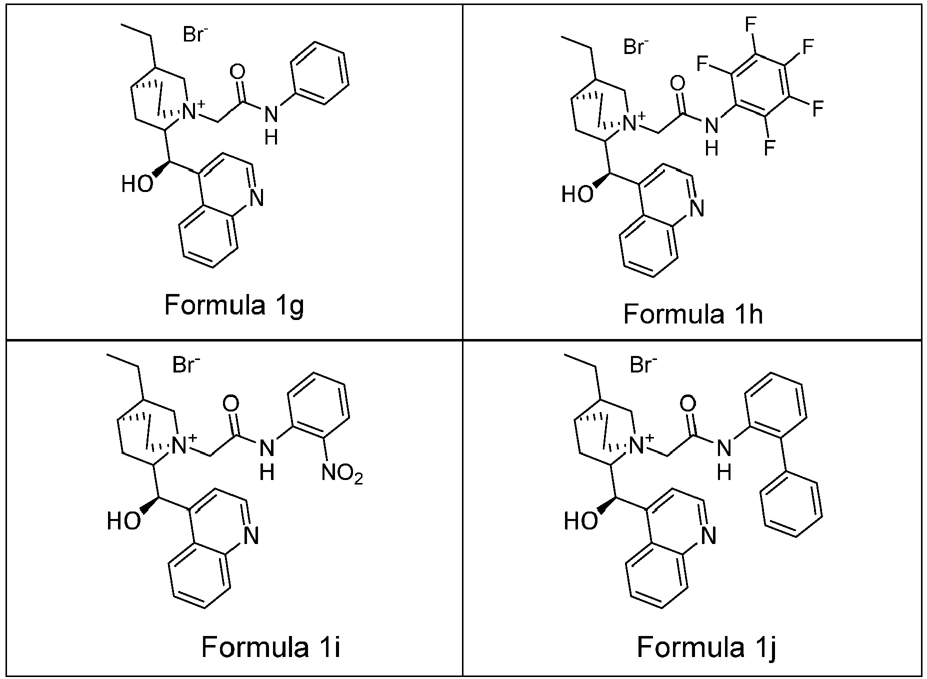

4. The compound according to claim 1, wherein the compound of Formula 1 is:

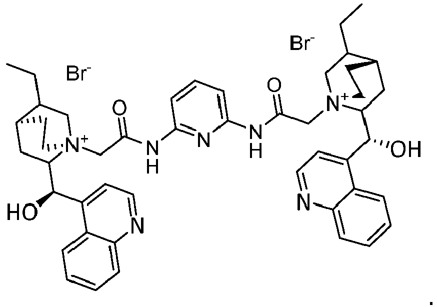

5. A method for preparation of a compound as defined in claim 2, the method comprising reacting two molar equivalents of a compound of formula: 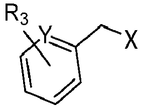 wherein:
**R₃** is a halogen atom (-F, -CI, -Br or -I), a trifluoromethyl (-CF₃), nitrile (-CN) or nitro (-NO₂) group;
Y is any heteroatom;
**X** is Cl or Br;
with a compound selected from the group of hydrocinchonidine, hydrocinchonine, hydroquinine or hydroquinidine, wherein the reaction is performed for 1 to 5 hours at room temperature in a two-phase system using dichloromethane as an aprotic solvent.

6. A method for preparation of a compound as defined in claim 3, the method comprising reacting no more than two molar equivalents of a compound of formula: 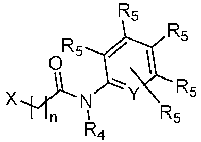 wherein:
**n** is an integer ranging from 1 to 5;
**R₄** is independently hydrogen, C₁-C₅ alkyl, or C₅-C₁₆ aryl;
**R₅** is independently hydrogen, halogen, C₁-C₁₂ alkyl, C₅-C₁₆ aryl, C₁-C₅ branched alkyl, nitro (-NO₂),
trifluoromethyl (-CF₃), nitrile (-CN), alkoxy -OC₁-C₁₂ group;
**X** is Cl or Br;
**Y** is a nitrogen atom or a CH group;
with a compound selected from the group of hydrocinchonidine, hydrocinchonine, hydroquinine or hydroquinidine, wherein the reaction is performed for 1 to 5 hours in an aprotic solvent at reflux temperature.

7. A method for preparation of a compound as defined in claim 4, the method comprising reacting no more than one molar equivalent of a compound of formula: 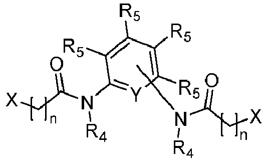 wherein:
**n** is an integer ranging from 1 to 5;
**R4** is independently hydrogen, C₁-C₅ alkyl, or C₅-C₁₆ aryl;
**R₅** is independently hydrogen, halogen, C₁-C₁₂ alkyl, C₅-C₁₆ aryl, C₁-C₅ branched alkyl, nitro (-NO₂), trifluoromethyl (-CF₃), nitrile (-CN), alkoxy -OC₁-C₁₂ group;
**X** is Cl or Br;
**Y** is a nitrogen atom or a CH group;
with a compound selected from the group of hydrocinchonidine, hydrocinchonine, hydroquinine or hydroquinidine, wherein the reaction is performed for 1 to 5 hours in an aprotic solvent at reflux temperature.

8. The use of a compound according to any one of claims 1 to 4 as a catalyst in an asymmetric phase transfer reaction (PTC).

9. The use according to claim 8, wherein said asymmetric reaction is an asymmetric Schiff base alkylation reaction, an asymmetric epoxidation reaction, or an asymmetric Michael addition reaction.

10. The use according to claim 9, wherein said asymmetric Michael addition reaction is an asymmetric cyanation reaction.

11. The use according to claim 9, wherein said asymmetric Schiff base alkylation reaction takes place with imine derivatives of α-amino acid esters as substrates.

12. The use according to any one of claims 8 to 11, wherein the reaction takes place under liquid-liquid or liquid-solid phase transfer conditions.

13. The use according to any one of claims 8 to 12, wherein the reaction is carried out under flow conditions.

14. The use of a compound as defined in claim 2 as a catalyst in an asymmetric phase transfer reaction (PTC) wherein the catalyst is generated *in situ* or the catalyzed reaction is carried out using a one-pot technique.
